# EUROPEAN PATENT APPLICATION

(11) **EP 1 281 754 A1**
(43) Date of publication of application: **05.02.2003**
(21) Application number: 01930018.5
(22) Date of filing: 10.05.2001
(51) Int. Cl.: C12N 5/06, C12N 5/08, C12N 5/10, C12Q 1/70, A61K 45/00, A61P 31/18, G01N 33/50, G01N 33/15

(54) **HIV-PRODUCING CELL LINE AND USE THEREOF**

(30) Priority: 11.05.2000 JP 2000143416
(71) Applicant: Takeda Chemical Industries, Ltd., Osaka-shi, Osaka 541-0045 (JP)
(72) Inventor: MIYAKE, Hiroshi, Kagoshima-shi Kagoshima 891-0175 (JP); IIZAWA, Yuji, Mukou-shi Kyoto 617-0002 (JP); BABA, Masanori, Kagoshima-shi Kagoshima 891-0103 (JP)
(74) Representative: Lewin, John Harvey
(86) International application number: JP0103887
(87) International publication number: WO01085919

(57) **Abstract**

The object of the present invention is to provide a T cell line capable of continuously producing human immunodeficiency virus (HIV) using CC chemokine receptor 5 (CCR5); and being in a state of continuously producing human immunodeficiency virus (HIV) or in a state wherein human immunodeficiency virus (HIV) is latently infecting, and a method for isolating and producing the T cell line.

The present invention related to T cell line capable continuously producing human immunodeficiency virus (HIV) using cc chemokine receptor 5 (CCR5) and being in a state of continuously producing human immunodeficiency virus (HIV) is latently infecting, a method for screenings by use of the T cell line of (1) HIV resistant to an agent having an anti-HIV activity, (2) an agent having an anti-HIV activity, (3) an agent inhibiting the transition of HIV from the latent period into the proliferation period, and (4) an agent capable of eliminating HIV from T cell line; and a pharmaceutical composition comprising the agent obtained by the above screenings of (2) to (4).

A T cell line of the present invention having a continuous productivity of human immunodeficiency virus (HIV) usnig CC Chemokine receptor 5 (CCR5) is useful in screening for agents or salts thereof for the treatment or prevention of diseases such as AIDS caused by HIV.

## Description

### Technical Field

The present invention relates to (1) a novel T cell line (preferably human T cell line) capable of continuously producing a human immunodeficiency virus (HIV) using CC chemokine receptor 5 (CCR5); (2) a method for preparing said cell line; (3) a method for preparing (i) HIV using CCR5, or (ii) HIV resistant to an agent having an anti-HIV activity, which comprises using said cell line; (4) a method for screening an agent (i) having an anti-HIV activity, (ii) inhibiting transition of HIV from latent period to proliferation period, or (iii) eliminating HIV from a T cell line, which comprises using said cell line; and (5) an agent obtained by use of said screening method (i) having an anti-HIV activity, (ii) inhibiting transition of HIV from latent period to proliferation period, or (iii) eliminating HIV from a T cell line; and so on.

### Background Art

A human immunodeficiency virus (HIV) is a retrovirus in which its own genetic information is encoded in its ribonucleic acid (RNA), and known as a causative virus of acquired immunodeficiency syndrome (AIDS). HIV is classified into two groups according to difference in the nucleotide sequences. Of these, because there are still more patient infected with human immunodeficiency virus type 1 (HIV-1) than those infected with human immunodeficiency virus type 2 (HIV-2), HIV-1 has been specifically studied, and its life-cycle has been understood in detail. HIV-1 infects cells through CD4 (cluster of differentiation 4) and a chemokine receptor, and converts its genetic information from RNA to deoxyribonucleic acid (DNA) using its own reverse transcriptase, and then it is integrated into genome DNA of the host cell to become a provirus. Provirus makes enzymes of the host cell to transcript and translate its sequence, and finally a large number of mature virus particles are formed and they bud from the host cell. HIV-1 proliferates by repeat this life-cycle of infection, reverse transcription, integration, formation of particles and budding. However because accuracy of reverse transcriptional reaction is low, the nucleotide sequence of the budded virus often differs from that of the HIV-1 initially infected. Change of nucleotide sequence results in variety of HIV-1, which causes a HIV-1 to which an anti-HIV-1 agent is not effective, as mentioned below. Screening has been widely done for an agent inhibiting HIV, in particular proliferation of HIV-1 (an anti-HIV-1 agent) because such an agent prevents development of AIDS in a patient infected with HIV-1. Up to now, an inhibitor of reverse transcriptase and an inhibitor of proteases (protease inhibitor) derived from HIV-1 involving in formation of particles have been actually administered to patients infected with HIV-1, and have been proved to be effective. However as mentioned above, because there are polymorphism in HIV-1, it is possible that HIV-1 emerges to which each anti-HIV-1 agent is not effective, and many HIV-1 have been isolated which are resistant to agents actually used clinically. It is possible that HIV-1 emerges to which no current agents are effective. Screening for novel anti-HIV-1 agents is actively carried out.

HIV-1 is largely classified into two groups according to their chemokine receptor usage in infection: viruses using CD4 and CC chemokine receptor 5 (CCR5) in infection is CCR5 using (R5) HIV-1; and viruses using CD4 and CXC chemokine receptor 4 (CXCR4) in infection is CXCR4 using (X4) HIV-1. After infection of HIV-1, asymptomatic period lasts for several years to ten and several years, then AIDS develops. It is known that R5 HIV-1 is mainly isolated in asymptomatic period and that X4 HIV-1 is mainly isolated in AIDS patients. Because it is also known that HIV-1 actively proliferates during asymptomatic period, it is thought that development of AIDS can be prevented more effectively by inhibiting proliferation of HIV-1 in this period. Therefore screening for agents inhibiting proliferation of R5 HIV-1 is widely and extensively carried out. Also, in order to eradicate HIV-1 from patient's body, only inhibition of proliferation of HIV-1 is not sufficient, and it is necessary to eliminate proviruses integrated into genome DNA of the host. However current anti-HIV-1 agents are not effective to eliminate proviruses. It is necessary to develop anti-HIV-1 agents which have novel mechanism and can eliminate proviruses.

However cell lines generally cultured in laboratories do not express a sufficient amount of CCR5, and infection with R5 HIV-1 is not sufficient (Lijun Wu et al., Journal of Experimental Medicine, 185, 1681-1691 (1997)). Because proliferation of HIV-1 varies depending on blood donors for preparing peripheral blood mononuclear cells, reproducibility of infection experiments is not so high when preparation of R5 HIV-1 and various infection experiments are carried out using peripheral blood mononuclear cells. This inhibits effective screening for anti-R5 HIV-1 agents. Moreover when peripheral blood mononuclear cells are used, there are problems of unstable yield in preparation of R5 HIV-1, and decrease in reproducibility of results of experiments due to polymorphism of the virus caused by repeated infection for preparation of R5 HIV-1 and accumulation of mutation in nucleotide sequence. Also, in order to screen for an agent which eliminate provirus of R5 HIV-1, a T cell line is required in which R5 HIV-1 is integrated into its genome DNA and which is in a state of continuously producing virus particles or in a latent state. However T cell line which is chronically infected with R5 HIV-1 has not been reported.

If a subclonable and immortalized T cell line in which R5 HIV-1 is integrated into its genome DNA, and which is capable of continuously producing virus or which is in latent state, is established, it can be used in screening for an agent which eliminates proviruses. Furthermore, highly reproducible screening can be carried out for an agent which inhibits proliferation of R5 HIV-1 because viruses with same nucleotide sequence can be stably prepared from such T cell line. By screening in a high reproducibility, possibility of discovering an anti-R5 HIV-1 agent increases, and huge benefit can be presented to pharmaceutical field. Therefore, it is necessary to present a T cell line capable of continuously producing human immunodeficiency virus (HIV) using CC chemokine receptor 5 (CCR5), or a T cell line which is in a state wherein R5 HIV-1 is latently infecting, and to isolate and produce said T cell line, and to present a screening method using said T cell line.

### Disclosure of Invention

The inventors have eagerly studied taking the above problems into consideration. The inventors firstly succeeded in establishment of a subclonable and immortalized T cell line capable of continuously producing human immunodeficiency virus (HIV) using CC chemokine receptor 5 (CCR5), or a subclonable and immortalized T cell line wherein HIV is in latently infecting. At last the present invention is completed.

That is, the present invention relates to:
(1) a T cell line capable of continuously producing human immunodeficiency virus using CC chemokine receptor 5;
(2) the T cell line described in (1) which is in a state of continuously producing human immunodeficiency virus using CC chemokine receptor 5;
(3) the T cell line described in (1) which is in a state wherein human immunodeficiency virus using CC chemokine receptor 5 is latently infecting;
(4) the T cell line described in (1) which is MOLT-4 cell line;
(5) the T cell line described in (1) wherein human immunodeficiency virus using CC chemokine receptor 5 is human immunodeficiency virus type 1 using CC chemokine receptor 5;
(6) the T cell line described in (1) wherein human immunodeficiency virus using CC chemokine receptor 5 is HIV-1 Ba-L;
(7) a method of preparing the T cell line described in (1), which comprises infecting a T cell line capable of being chronically infected with human immunodeficiency virus using CC chemokine receptor 5, with human immunodeficiency virus (HIV);
(8) the method described in (7) wherein a T cell line capable of being chronically infected with human immunodeficiency virus using CC chemokine receptor 5 is MOLT-4/CCR5 (FERM BP-7060);
(9) a method for preparing human immunodeficiency virus (HIV), which comprises culturing a T cell line which is in a state of continuously producing human immunodeficiency virus using CC chemokine receptor 5;
(10) a method for screening human immunodeficiency virus (HIV) resistant to an agent, which comprises culturing in the presence of said agent a T cell line which is in a state of continuously producing human immunodeficiency virus using CC chemokine receptor 5;
(11) a method for screening an agent having an antihuman immunodeficiency virus (HIV) activity, which comprises using the T cell line described in (2);
(12) a method for screening an agent which inhibits transition of human immunodeficiency virus (HIV) from latent infection period to proliferation period, which comprises using the T cell line described in (3);
(13) a method for screening an agent which eliminates human immunodeficiency virus (HIV) from a T cell line, which comprises using the T cell line described in (1);
(14) an agent which is obtained by the screening method described in (11);
(15) an agent which is obtained by the screening method described in (12);
(16) an agent which is obtained by the screening method described in (13);
(17) a pharmaceutical composition comprising the agent described in (14), (15) or (16); and
(18) the pharmaceutical composition described in (17) which is an anti-AIDS pharmaceutical composition.

### Brief Description of Drawings

Figure 1 shows a chart of flow cytometry of MOLT4 cell line. The solid line represents the control antibody, and the broken line represents the CCR5 antibody.
Figure 2 shows a chart of flow cytometry of MOLT4/CCR5 cell line. The solid line represents the control antibody, and the broken line represents the CCR5 antibody.

### Best Mode for Carrying Out the Invention

"Human immunodeficiency virus using CC chemokine receptor 5 (CCR5)" in the specification means human immunodeficiency virus which requires (is directed to) CC chemokine receptor 5 in infection to cell line. A preferable example is human immunodeficiency virus type 1 which requires CCR5 in infection to cell line. Human immunodeficiency virus type 1 Ba-L is particularly preferable.

"T cell line capable of continuously producing human immunodeficiency virus using CC chemokine receptor 5" includes both a T cell line which is in a state of continuously producing human immunodeficiency virus using CCR5 and a T cell line which is in a state wherein human immunodeficiency virus using CCR5 is latently infecting. "T cell line which is in a state wherein HIV is latently infecting" means that HIV is integrated into genome DNA of the T cell line but that the T cell line does not produce mature virus particles.

A preferable example of T cell line is MOLT-4 cell line described in Journal of the National Cancer Institute, 49, 891-895 (1972).

T cell line of the present invention capable of continuously producing human immunodeficiency virus using CCR5 can be prepared by infecting a T cell line capable of being chronically infected with human immunodeficiency virus using CCR5, with human immunodeficiency virus using CCR5. "T cell line capable of being chronically infected with human immunodeficiency virus using CCR5" in the present specification means a T cell line expressing sufficient CD4 and CCR5 which are receptors of human immunodeficiency virus using CCR5. Here, an example of a raw material of T cell line capable of being chronically infected with human immunodeficiency virus using CCR5 is an immortalized T cell line obtained by cloning a malignantly altered T cell such as a human leukemia T cell. Cloning can be done according to various known methods. Concretely, an example of such a method is to cloning an immortalized cell from a human leukemia T cell line, from viewpoint of permanent proliferation of tumor tissues. An example of the immortalized T cell line thus established include, but not limited to, MOLT-4 Clone 8 cell line (Journal of Virology, pp.1159-1162, March, 1986). The immortalized T cell line thus obtained can be transformed by a known method, and a T cell line capable of being chronically infected with human immunodeficiency virus using CCR5 can be produced by expression of CC chemokine receptor 5 gene in said T cell line. Concretely, said immortalized T cell line is transformed using plasmid pcDNA3.1-CCR5 described in Proceedings of the National Academy of Sciences USA, 96, 5698-5703 (1999). Said transformation is done by a known method, for example a general method by Sambrook et al., Molecular Cloning: A Laboratory Manual, Cold Spring Harbor Laboratory Press. A preferable example of a T cell line capable of being chronically infected with human immunodeficiency virus using CCR5 is MOLT-4/CCR5 (FERM BP-7060) cell line obtained in Example 1 described below.

T cell line of the present invention capable of continuously producing human immunodeficiency virus using CCR5 can be prepared by infecting a T cell line capable of being chronically infected with human immunodeficiency virus using CCR5, with human immunodeficiency virus using CCR5. Infection of human immunodeficiency virus is carried out by culturing a T cell line capable of being chronically infected with human immunodeficiency virus using CCR5 with human immunodeficiency virus using CCR5, for example in a medium used for culture of T cell lines (e.g. RPMI 1640 medium, etc). Culture temperature is between about 15 °C and about 55°C, preferably between about 20°C and about 45°C, more preferably between about 25°C and about 40°C. Culture period is about 1 hour to about 6 months, preferably about 1.5 hours to about 60 days. Preferably culture is done under carbon dioxide. Concentration of carbon dioxide is about 3% (v/v) to 10% (v/v).

Completion of infection with human immunodeficiency virus can be detected by measuring the increase of an amount of HIV in the supernatant of T cell line culture. The amount of HIV can be measured by (1) determining HIV RNA by RT-PCR method, (2) determining p24 by enzyme-linked immunosorbent assay (for example, using Retro-Tek HIV-1 p24 Antigen ELISA kit, Zepto Metrix, USA), or (3) determining HIV reverse transcriptase activity.

Using a T cell line of the present invention capable of continuously producing human immunodeficiency virus using CCR5, and an agent having an anti-HIV activity or a test substance, following screenings can be done: [1] screening for HIV which is resistant to said agent having an anti-HIV activity by culturing T cell line which is in a state of continuously producing human immunodeficiency virus (HIV) using CC chemokine receptor 5 (CCR5) in the presence of an agent having an anti-HIV activity, [2] screening for an agent having an anti-HIV activity using T cell line which is in a state of continuously producing human immunodeficiency virus (HIV) using CC chemokine receptor 5 (CCR5), [3] screening for an agent which inhibits transition of said HIV from latent period to proliferation period using a T cell line which is in a state wherein human immunodeficiency virus (HIV) using CC chemokine receptor 5 (CCR5) is latently infecting, and [4] screening for an agent which eradicates said HIV from a T cell line using a T cell line capable of producing human immunodeficiency virus (HIV) using CC chemokine receptor 5 (CCR5).

Examples of agents having an anti-HIV activity include, but not to limited to, anti-HIV agents such as an inhibitor of reverse transcriptase and a protease inhibitor.

Examples of the test substances having an anti-HIV activity include, but not to limited to, peptides, proteins, non-peptide compounds, synthesized compounds, fermented products, cell extracts, plant extracts, animal extracts, and sera. These substances may be prepared by a known method or a similar method thereto.

Next, each screening method is described:
[1] screening for HIV which is resistant to said agent having an anti-HIV activity by culturing T cell line which is in a state of continuously producing human immunodeficiency virus (HIV) using CC chemokine receptor 5 (CCR5) in the presence of an agent having an anti-HIV activity.
   A T cell line continuously producing HIV using CCR5 or its culture supernatant is cultured with cells to which said HIV can infect (for example, a T cell line capable of being chronically infected with HIV using CCR5 as above described and peripheral blood mononuclear cells) in the presence of an agent having an anti-HIV activity. Culture conditions are those as described above. Proliferated HIV in the culture supernatant is stepwise diluted, mixed with cells to which said HIV can infect, and cultured as above. HIV is isolated from the culture supernatant and/or the infected cells by a known method. HIV resistant to said agent having an anti-HIV activity can be screened by culturing the cloned HIV isolated under the similar condition wherein said cells to which HIV can infect and said agent having an anti-HIV activity are exist, and identifying proliferation of HIV.
[2] screening for an agent having an anti-HIV activity using T cell line which is in a state of continuously producing human immunodeficiency virus (HIV) using CC chemokine receptor 5 (CCR5).
   A T cell line in a state of continuously producing HIV using CCR5 or its culture supernatant is cultured with said cells capable of being infected with HIV in the presence of test substance under the conditions above described. Screening can be done for an agent having an anti-HIV activity by measuring the amount of HIV in the culture supernatant using the method above described, and selecting an agent inhibiting increase of HIV amount.
[3] screening for an agent which inhibits transition of said HIV from latent period to proliferation period using a T cell line which is in a state wherein human immunodeficiency virus (HIV) using CC chemokine receptor 5 (CCR5) is latently infecting.
   A T cell line which is in a state wherein HIV using CCR5 is latently infecting is cultured under similar conditions described above, in the presence of an agent which leads HIV-producing period (proliferation period) (for example, an agent such as TNF-a which activates HIV transcription via LTR (long terminal repeat) sequence of HIV in a state of latent infection) and a test substance. Screening for an agent can be done which inhibits transition of HIV from latent period to proliferation period by measuring the amount of HIV in the culture supernatant is measured by a method as above described, and selecting the test substance when HIV amount is not detected or low.
[4] screening for an agent which eliminates said HIV from a T cell line using a T cell line capable of producing human immunodeficiency virus (HIV) using CC chemokine receptor 5 (CCR5).

T cell line capable of producing HIV using CCR5 is cultured in the presence of a test compound under similar conditions as above mentioned. An agent which eliminates said HIV from a T cell lines can be screened by measuring an amount of provirus DNA of a T cell line capable of producing HIV using CCR5 by PCR method or Southern hybridization method, and selecting an agent which decrease the amount of provirus.

In order to measure the amount of provirus DNA by PCR method, for example, genome DNA is extracted from cultured T cell line by a method known in the art, a quantitative PCR reaction is carried out by a known method using genome DNA extracted as a template, any region of said provirus DNA is amplified, and then the genome DNA amount is measured by applying a fluorescent-labeled or radio-labeled probe, or a dye which binds DNA (for example ethidium bromide) to the amplified DNA fragment.

In order to measure the amount of provirus DNA by Southern hybridization method, for example genome DNA is extracted from cultured T cell line by a method known in the art, and the genome DNA amount is measured by Southern hybridization by a known method using extracted DNA and a probe (for example, a fluorescent-labeled probe specific to HIV, or a radio-labeled probe specific to HIV).

HIV obtained by the screening method [1] of the present invention, which is resistant to an agent having an anti-HIV activity, can be used to investigate the resistance mechanism by use of the resistant HIV, and to search agents effective to the resistant HIV.

Agents obtained by the screening methods [2]-[4] of the present invention are those selected from the test substances above mentioned (for example, peptides, proteins, non-peptide compounds, synthetic compounds, fermented products, cell extracts, plant extracts, animal extracts, sera, etc). Because they have preventive and/or therapeutic effects to diseases resulting from HIV (for example AIDS), they can be used as safe and low-toxic pharmaceuticals for prevention and/or treatment of said diseases. In addition, agents which are derived from the agents obtained by the screenings [2]-[4] can be also used likewise. These agents or the agents derived from them may be used solely for treatment and/or prevention of diseases, or they may be used in combination with other anti-HIV agents (for example, nucleoside reverse transcriptase inhibitors such as azidothymidine and dideoxyinosine; non-nucleoside reverse transcriptase inhibitors such as nevirapine; HIV protease inhibitors such as indinavir, saquinavir, ritonavir and nelfinavir).

Agents obtained by said screening methods include salts thereof when they form salts. Salts, for example those with physiologically acceptable acids (e.g. inorganic acids, organic acids) or with bases (e.g. alkali metals, organic amines) can be used. Particularly, physiologically acceptable acid addition salts are preferable. Examples of such salts include, but not limited to, salts with inorganic acids (e.g. hydrochloric acid, phosphoric acid, hydrobromic acid, sulfuric acid), and salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid).

A pharmaceutical composition comprising an agent obtained by said screening can be prepared by methods known in the art or similar methods. The parmaceutical composition thus obtained is safe and low-toxic, therefore it can be administered to human being or mammals (for example, rat, mouse, guinea pig, rabbit, sheep, pig, bovine, horse, cat, dog, monkey, etc).

Dose of the agent obtained by said screening method varies depending on disease to be treated, subject, mode of administration, etc, and generally about 0.1mg to 5g, preferably about 1.0mg to 4.5g, more preferably about 2mg to 4g of the agent or a salt thereof per day is administered orally to an adult (60kg) to treat AIDS. In case of parenteral administration, unit dose of said agent varies depending on subject, disease, etc, and for example, generally about 0.01mg to 35mg, preferably about 0.1mg to 20mg, more preferably about 0.1mg to 10mg per day can be administrated, preferably in a form of injection (e.g. intravenous injection, intramuscular injection, subcutaneous injection) to an adult (60kg) for treatment of AIDS. When the agent is administered to other animals, the amount corresponding to that administered to a 60kg adult can be given.

Examples of dosage forms of the pharmaceutical compositions above described includes, but not limited to, tablets (including sugar-coated tablets, film-coated tablets), pills, capsules (including microcapsules), granules, finely-divided granules, powders, syrups, emulsions, injections, inhalants, ointments, suppositories. These formulations can be prepared according to by known methods (for example methods described in Japanese Pharmacopoeia).

In said formulations, amount of the agent obtained by said screening method varies depending on forms of the pharmaceutical composition, and generally is about 0.01 to 100 weight %, preferably about 0.1 to 60 weight %, more preferably about 0.5 to 20 weight % to whole composition.

Concretely, a tablet can be prepared by granulating the agent obtained by the screening method of the invention solely or by granulating the agent mixed homogeneously with excipient, binder, disintegrator or appropriate additives by a conventional method, and then compressing the granule with addition of lubricant; or by directly compressing the agent solely or direct compressing the agent mixed homogeneously with excipient, binder, disintegrator or appropriate additives; or by compressing granules previously prepared solely, or mixing the granules homogeneously with appropriate additives, and then compressing them. As appropriate, colorant, corrigant, etc can be added to the pharmaceutical composition of the present invention.

As for an injection, an aliquot of the agent obtained by the screening method of the invention is solved, suspended or emulsified in water for injection, isotonic sodium chloride solution, Ringer's solution, etc (in case of an aqueous solvent) or, in general, vegetable oils, etc (in case of non-aqueous solvent), and then a certain amount of injection can be obtained. Alternatively, an aliquot of the agent is put into a vessel for injection (for example an ampoule), and sealed.

A suppository can be prepared by mixing the agent obtained by the screening method of the invention with a non-stimulating base, for example cocoa butter or polyethylene glycols that is solid at room temperature, and liquid at a temperature of intestinal tract and release the agent.

As a carrier for parenteral composition, a material conventionally used in pharmaceutical field, for example starch, mannitol, crystalline cellulose, sodium carboxymethyl cellulose, etc can be used. As a carrier for injection, for example distilled water, isotonic sodium chloride solution, glucose solution, transfusion formulation, etc can be used. As appropriate, additives generally used for pharmaceutical composition can be added, if necessary.

MOT-4/CCR5 cell line obtained in Example 1 described below was deposited to National Institute of Bioscience and Human-Technology, Agency of Industrial Science & Technology, Ministry of International Trade & Industry (NIBH) on February 29, 2000, and assigned an accession number, FERM BP-7060. The cell line was also deposited to Institute for Fermentation, Osaka (IFO) on January 27, 2000, and assigned an accession number, IFO 50521.

### Examples

Examples of the present invention are described below. The present is not construed to be limited to the Examples. Unless specifically described, procedures for gene manipulation are general procedures described Sambrook et al., Molecular Cloning: A Laboratory Manual, Cold Spring Harbor Laboratory Press.

### Example 1: Establishment of MOLT-4/CCR5 cell line

### (1) determination of a concentration of selection agent (Geneticin)

MOLT-4 cell was cultured at 37 °C under 5% CO₂ using RPMI1640 medium (lifetechnology, USA) containing 10% fetal bovine serum (FBS, ICN, U.S.A.), 100 U/ml of penicillin G (lifetechnology, USA), 100 µg / ml of streptomycin (lifetechnology, USA)(thereafter referred to "MOLT-4 basal medium").

Cells were cultured at 2x10³ cells/ml in MOLT-4 basal medium containing 100, 200, 300, 400, 500, 600, 700, 800, 900, 1000 µg/ml of Geneticin (lifetechnology, USA)for two weeks and observed under a microscope. The minimum concentration of complete inhibition of cell proliferation was determined.

### (2) Transfection

15µl of Cellfectin (lifetechnology, USA) was added to 250 µl of RPMI1640 medium without FBS, penicillin G, and streptomycin to prepare a lipid solution, and the lipid solution was added to a 24-well plate.

Likewise, 3.2 µg of pcDNA3.1-CCR5 (Baba, et al, Proceedings of the National Academy of Sciences , USA, 1999, vol. 96 5698-5703) was added to 250 µl of RPMI1640 medium containing no supplements to prepare a DNA solution. This DNA solution and the lipid solution was mixed together at room temperature for 30 minutes. Then, 250 µl of DNA-lipid solution was removed from A1 well.

50 µl of MOLT-4 cell suspension was added to RPMI1640 medium containing no supplements to a density of 8x10 ⁶ cells/ml, mixed with DNA-lipid solution and then cultured. at 37°C under 5% CO₂ for 4 hours. Further, 600 µl of RPMI1640 medium containing 15% FBS, 100 U/ml penicillin G and 100 µg/ml streptomycin was added, and cultured for 2 days. Whole the culture was transferred to a 100mm culture dish containing 10ml of MOLT-4 basal medium with 1mg/ml Geneticin, and cultured at 37°C under 5% CO₂ with changing the culture medium at appropriate times to proliferate Geneticin-resistant cells.

### (3) Preparation of feeder cell

MOLT-4 cell was suspended in a phosphate buffer solution (PBS (lifetechnology, USA) ) to a density of 1x10⁷ cells/ml. 200µl of the solution was transferred into each sample tube. 20µl of 0.5 mg/ml mitomycin C solution (Wako Pure Chemical Industries, Ltd., Japan) was added thereto and incubated at 37°C for 30 minutes. The cell was washed five times with MOLT-4 basal medium then it was suspended in MOLT-4 basal medium containing 1 mg/ml of Geneticin to a density of 1x10⁵ cells/ml.

### (4) Single cell cloning

100 µl of the feeder cells above described were transferred to a 96-well plate. Geneticin resistant cells proliferated were suspended in MOLT-4 basal medium containing 1mg/ml Geneticin to a density of 3 cells/ml. Then, they were transferred into the 96-well plate to which the feeder cells previously had been added. The proliferation of cells was observed under a microscope. The proliferating cells were isolated, and the scale of the culture was made larger at appropriate times.

### (5) Examination of CCR5 expression

The Geneticin-resistant clones obtained according to (4) above were washed twice with a reaction solution [2%(V/V)FBS, 0.1%(W/V) sodium azide (Wako Pure Chemical Industries, Ltd., Japan)/PBS]. They were suspended in 40 µl of the same reaction solution. 10 µl of fluorescein isothiocyanate (FITC)-labeled anti-CCR5 antibody clone 2D7 (Farmingen, U.S.A) was added and reacted at 4 °C for 45 minutes.

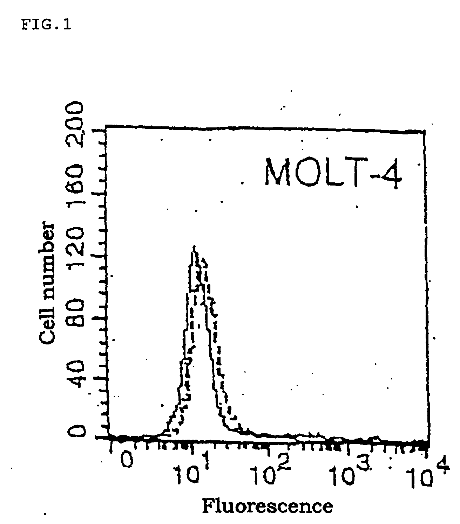

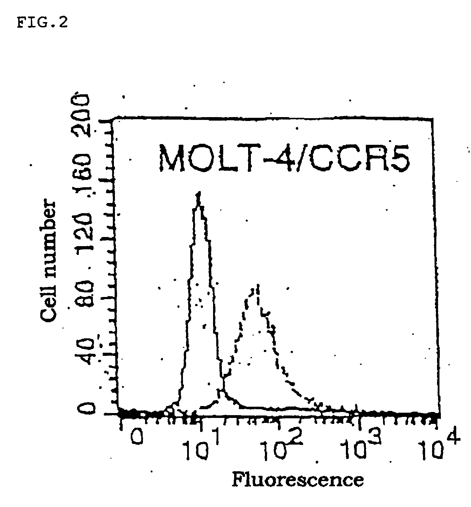

The cells were separated and washed three times with 1 ml of each reaction solution above described, and then suspended in 300µl of the reaction solution. The binding of antibody was analyzed by Flow Cytometer (JASCO CytoACE-300, Nippon Bunko, Ltd., Japan). The result was shown in Figure 1 and Figure 2.

Comparing the Flow Cytometer chart (Figure 2) of MOLT4/CCR5 cell line with the Flow Cytometer chart (Figure 1) of MOLT4 cell line which is a raw material, the Flow Cytometer chart (Figure 2) of MOLT4/CCR5 cell line showed a clear shift to right (to high fluorescence intensity) of the peak of anti-CCR5 antibody (broken line). As a result, the expression of CCR5 is confirmed.

### Example 2: Establishment of MOLT-4/CCR5/Ba-L cell line

### (1) Infection of HIV-1 Ba-L

The MOLT-4/CCR5 cell line obtained in Example 1 was cultured in MOLT-4 basal medium at 37 under 5% CO₂. HIV-1 Ba-L (an amount of which is equivalent to 10 ng of p24) was added to MOLT-4/CCR5 cell prepared to be a concentration of 1x10⁶ cells/ml in MOLT-4 basal medium, and incubated at 37°C under 5% CO₂ for 2 hours.

The cells were washed with MOLT-4 basal mediumto remove unadsorbed viral particles. After the washing, the cells were re-prepared to concentration of 1x10⁵ cells/ml and incubated at 37°C under 5% CO₂.

### (2) Establishment of continuous infection cell line

A supernatant of HIV-1 Ba-L infected MOLT-4/ CCR5 cell line obtained from above (1) was collected every 4 days. At the same time the number of cells was determined by trypane blue staining. Cells were re-suspended in MOLT-4 basal medium to a concentration of 1x10⁵ cells/ml and incubation was continued at 37°C under 5% CO₂. An amount of the p 24 antigen in the culture supernatant was measured using Retro-Tek HIV-1 p24 Antigen ELISA kit (Zepto Metrix, U.S.A.). The result was shown in Table 1. Incubation was continued, and most of MOLT-4/CCR5 cells died. However, after the incubation for a month, some of the cells survived and proliferated. The amount of p24 antigen in a supernatant of the medium was measured, and p24 antigen production was confirmed and a chronically infected cell line (MOLT-4/CCR5/Ba-L) was established. Single cell cloning of the chronically infected cell line was done by limited-dilution. As a result, the clone which was lost the production of p24 was obtained. By incubating the clone in a medium containing 10 ng/ml TNF- α for 3 days, p24 antigen can be detected in the supernatant of the medium and establishment of a latent infected cell line was confirmed.

**Table 1**

| Days cultured | Amount of p24 antigen(ng/ml) |
|---|---|
| 4 | 0.07±0.048 |
| 8 | 6.9±4.8 |
| 12 | 108±46 |

### INDUSTRIAL APPLICABILITY

A T cell line of the present invention having a continuous productivity of human immunodeficiency virus (HIV) using CC Chemokine receptor 5 (CCR5) is useful in screening for agents or salts thereof for the treatment or prevention of diseases such as AIDS caused by HIV.

## Claims

1. A T cell line capable of continuously producing human immunodeficiency virus using CC chemokine receptor 5.

2. The T cell line according to claim 1 which is in a state of continuously producing human immunodeficiency virus using CC chemokine receptor 5.

3. The T cell line according to claim 1 which is in a state wherein human immunodeficiency virus using CC chemokine receptor 5 is latently infecting.

4. The T cell line according to claim 1 which is MOLT-4 cell line.

5. The T cell line according to claim 1 wherein human immunodeficiency virus using CC chemokine receptor 5 is human immunodeficiency virus type 1 using CC chemokine receptor 5.

6. The T cell line according to claim 1 wherein human immunodeficiency virus using CC chemokine receptor 5 is HIV-1 Ba-L.

7. A method of preparing the T cell line according to claim 1, which comprises infecting a T cell line capable of being chronically infected with human immunodeficiency virus using CC chemokine receptor 5, with human immunodeficiency virus (HIV).

8. The method according to claim 7 wherein a T cell line capable of being chronically infected with human immunodeficiency virus using CC chemokine receptor 5 is MOLT-4/CCR5 (FERM BP-7060).

9. A method for preparing human immunodeficiency virus (HIV), which comprises culturing a T cell line which is in a state of continuously producing human immunodeficiency virus using CC chemokine receptor 5.

10. A method for screening human immunodeficiency virus (HIV) resistant to an agent, which comprises culturing in the presence of said agent a T cell line which is in a state of continuously producing human immunodeficiency virus using CC chemokine receptor 5.

11. A method for screening an agent having an antihuman immunodeficiency virus (HIV) activity, which comprises using the T cell line according to claim 2.

12. A method for screening an agent which inhibits transition of human immunodeficiency virus (HIV) from latent infection period to proliferation period, which comprises using the T cell line according to claim 3.

13. A method for screening an agent which eliminates human immunodeficiency virus (HIV) from a T cell line, which comprises using the T cell line according to claim 1.

14. An agent which is obtained by the screening method according to claim 11.

15. An agent which is obtained by the screening method according to claim 12.

16. An agent which is obtained by the screening method according to claim 13.

17. A pharmaceutical composition comprising the agent according to any one claim of claims 14, 15 and 16.

18. The pharmaceutical composition according to claim 17 which is an anti-AIDS pharmaceutical composition.
